# EUROPEAN PATENT SPECIFICATION

(11) **EP 1 015 452 B1**
(45) Date of publication and mention of the grant of the patent: **23.07.2003**
(21) Application number: 98943333.9
(22) Date of filing: 21.08.1998
(51) Int. Cl.: C07D 477/06, C07D 477/14

(54) **OPTIONALLY PROTECTED 3-HYDROXYMETHYL CARBAPENEMS AND SYNTHESIS**
GEGEBENENFALLS GESCHÜTZTE 3-HYDROXYMETHYLCARBAPENEME UND IHRE SYNTHESE
3-HYDROXYMETHYL CARBAPENEMES EVENTUELLEMENT PROTEGES ET SYNTHESE

(30) Priority: 26.08.1997 US 56967 P; 09.01.1998 GB 9800459
(43) Date of publication of application: 05.07.2000
(73) Proprietor: MERCK & CO., INC., Rahway, New Jersey 07065 (US)
(72) Inventor: CHUNG, John, Y., Rahway, NJ 07065 (US); LEAZER, Johnnie, L., Jr., Rahway, NJ 07065 (US); YASUDA, Nobuyoshi,, Rahway, NJ 07065 (US); JENSEN, Mark, S., Rahway, NJ 07065 (US); WELLS, Kenneth, M., Rahway, NJ 07065 (US); YANG, Chunhua, Rahway, NJ 07065 (US)
(74) Representative: Horgan, James Michael Frederic
(86) International application number: US9817467
(87) International publication number: WO99010348

(56) References cited:
- EP-A2- 0 476 649
- T.A. RANO ET AL: "A highly efficient method for the preparation of 2-aryl substituted carbapenems exploiting a Pd(O) mediated cross-coupling reaction" TETRAHEDRON LETTERS, vol. 31, no. 20, 1990, pages 2853-2856, XP002167275 OXFORD GB
- N. YASUDA ET AL: "Practical synthesis of anti-methicillin-resistant staphylococcus aureus (MRSA) carbapenem L-742,728" JOURNAL OF ORGANIC CHEMISTRY, vol. 63, no. 16, 7 August 1998 (1998-08-07), pages 5438-5446, XP002167276 EASTON US
- J.K.STILLE Angew.Chem.Int.Ed.vol.25, 508 (1986)

## Description

The present invention relates to intermediate compounds useful in the synthesis of carbapenem antibiotics. The carbapenems derived from the present invention are useful against gram positive microorganisms, especially methicillin resistant Staphylococcus aureus (MRSA), methicillin resistant Staphylococcus epidermidis (MRSE), and methicillin resistant coagulase negative Staphylococci (MRCNS). There is an increasing need for carbapenems effective against such pathogens, as well as intermediates which facilitate their production. The intermediates of the present invention thus facilitate an important contribution to therapy for treating infections caused by these difficult to control pathogens.

Prior syntheses of similar intermediate compounds have relied on time-consuming, multi-step reaction protocols, and have generated the intermediate as an unstable oil. See, e.g., United States Patent No. 4,960,879, Shionogi & Co. Ltd. The present invention responds to the need for a more facile synthesis yielding novel, more stable carbapenern intermediates.

European Patent Publication No. 0 476 649 A (published 25^{th} March 1992) discloses a process for the preparation of carbapenem compounds.

T. A. Rano *et al, Tetrahedron Lett.,* **1990,** *31*, 2853-2856 discloses a process for the preparation of 2-aryl substituted carbapenem compounds via a palladium catalyzed cross-coupling reaction.

### SUMMARY OF THE INVENTION

The present invention relates to a process for preparation of a compound of formula I: 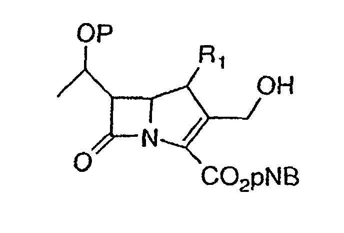 wherein:
R₁ represents CH₃ or H; and P represents a protecting group;
comprising reacting a compound of formula IV: 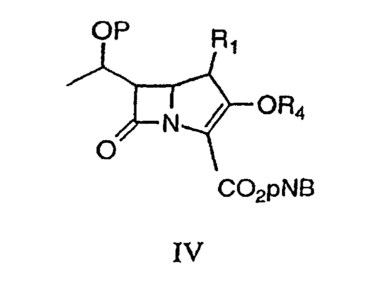 wherein R₁ and P and are defined above and R4 represents triflate or SO₂F;
in the presence of a catalyst and (R₃)₃SnCH₂OP", wherein : each R₃ represents C₁₋₄ lower alkyl, and P" represents H or a protecting group, to yield the compound of formula I.

This one-vessel process is a more efficient and facile pathway than the traditional multi-step synthesis for preparation of carbapenem intermediates.

The present invention also relates to the compound of formula I': 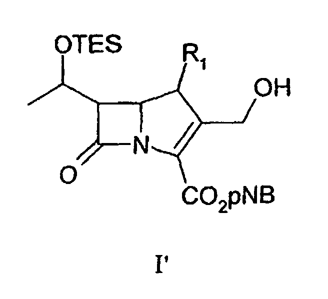 wherein R₁ represents CH₃ or H.

This novel compound is a stable and storable crystalline solid.

### BRIEF DESCRIPTION OF THE DRAWINGS

The invention is described in connection with the following drawings in which:
Fig. 1: X-ray crystallography pattern of compound Ia and
Fig. 2: Differential Scanning Calorimetric Cell (DSC) thermogram of compound Ia.

### DETAILED DESCRIPTION OF THE INVENTION

In one embodiment of the invention, a process for synthesizing a compound of formula I is described: 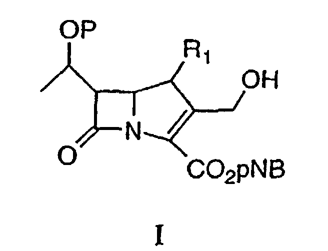 wherein:
wherein R₁ represents CH3 and P represents TES, TMS or TBS; comprising:
reacting a compound of formula IV: 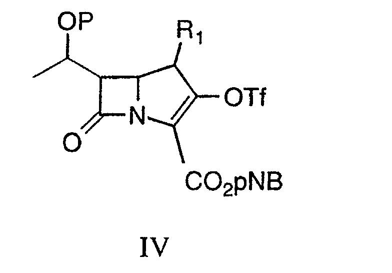 wherein P and R₁ are defined above; 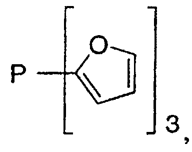 in the presence of ZnCl₂, Pd(dba)₂ ,
and
(R₃)₃SnCH₂OP", wherein :
R₃ represents a C₁₋₄ lower alkyl, and P" represents H, TES, TMS or TBS; to yield the compound of formula I.

In another embodiment of the invention, a process for synthesizing a compound of formula I is described: 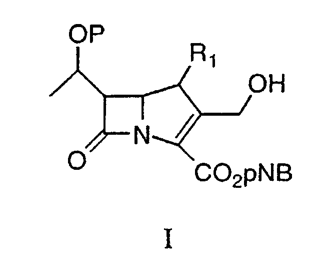 wherein R₁ represents CH₃ or H and P represents a protecting group, comprising:
step (1) heating a compound of formula II: 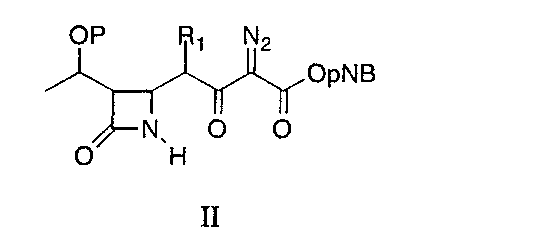 wherein R₁ and P are defined above;
in the presence of 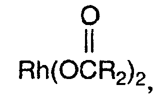 wherein R2 is C₁₋₁₅ alkyl, to yield a compound of formula III: 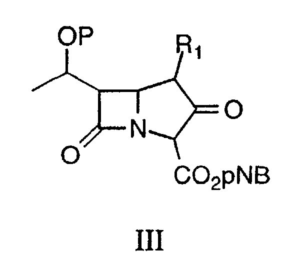 wherein R₁ and P are defined above;

Compound II is commercially available where P is H and R₁ is methyl or H. Compound II may also be synthesized according to the general methods disclosed in, for example, Nagao, et al., JOCEAH; J. Org. Chem., EN; 57; 15; 1992; 4243-4249; see also Schmitt, et al., J. Antibiotics 41(6):780-787 (1988).

A lewis acid may be added to the reaction mixture in this first step as an optional reagent. A preferred aspect of the invention is realized when a Lewis acid is employed. Suitable Lewis acids are described below.
step (2) reacting a compound of formula III with an amine, or combination of amines, and a trifluoromethanesulfonylation reagent or fluorosulfonylation reagent, to yield a compound of formula IV: 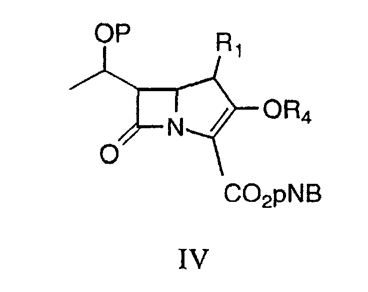 wherein R₁ and P are defined above and R₄ is Tf or SO₂F;
step (3) reacting a compound of formula IV in the presence of a catalyst, (R₃)₃SnCH₂OP", and optionally a phosphine ligand,
wherein : R₃ represents a C₁₋₄ lower alkyl, and P" represents H or a protecting group; to yield the compound of formula I. A preferred process is realized when a phosphine ligand is employed.

A Lewis acid may also be added to the reaction mixture at this third step as an optional reagent. A preferred process is realized when a Lewis acid is employed. Suitable Lewis acids for the first and third steps in the process include, independently, ZnBr₂, ZnCl₂, MgCl₂, MgBr₂, CaCl₂, and CaBr₂. Preferably, ZnCl₂ is used as the Lewis acid for both steps.

Some of the intermediates of the present invention occur as geometric isomers. The process of synthesizing all such isomers is encompassed by the present invention.

In a preferred aspect of this invention, R₁ is methyl and R₂ is C₁₋₁₂ alkyl.

In another preferred aspect of this invention a process for preparation of a compound of formula I: 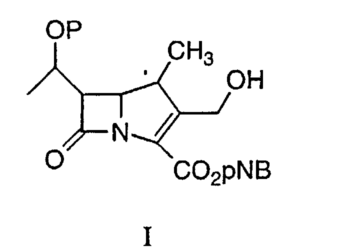 wherein P represents TMS, TES or TBS;
comprising refluxing a compound of formula II: 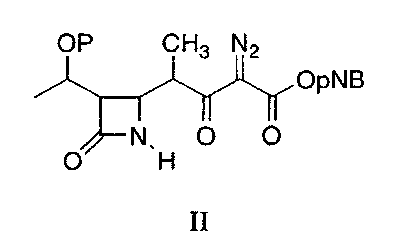 wherein P is defined above;
in the presence of ZnCl2 and 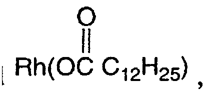 to yield a compound of formula III: 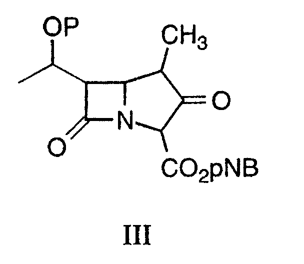 wherein P is defined above;
reacting a compound of formula III in the presence of DIEPA or 2,2,6,6-tetramethylpiperidine, or a combination thereof, and Tf20, to yield a compound of formula IV: 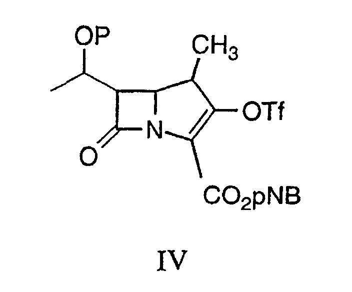 wherein P and is defined above;
reacting compound IV in the presence of a ZnCl₂, Pd(dba)₂ , 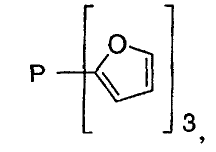 and (R₃)₃SnCH₂OP", wherein :
R3 represents a C₁₋₄ lower alkyl, and P" represents H, TMS, TES, or TBS;
to yield the compound of formula I is described.

The instant invention is generally carried out under an inert atmosphere using nitrogen or argon, preferrably argon.

The invention is described herein in detail, using the terms defined below unless otherwise specified.

pNB refers to the p-nitrobenzyl, represented by the following formula:

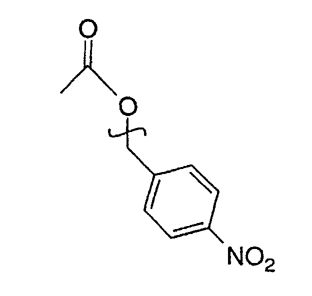

Tf refers to triflate (trifluoromethanesulfonyl).

When a functional group is termed "protected", this means that the group is in modified form to preclude undesired side reactions at the protected site. Suitable protecting groups for the compounds of the present invention will be recognized from the present application taking into account the level of skill in the art, and with reference to standard textbooks, such as Greene, T. W. et al. Protective Groups in Organic Synthesis Wiley, New York (1991). Examples of suitable protecting groups are: t-butylmethylphenylsilyl, t-butyldiphenylsilyl, trimethylsilyl (TMS), triethylsilyl (TES), t-butyldimethylsilyl (TBS), o-nitrobenzyloxycarbonyl, p-nitrobenzyloxycarbonyl (pNZ), p-nitrobenzyl (pNB), benzyloxycarbonyl, t-butyloxycarbonyl, 2,2,2-trichloroethyloxycarbonyl and allyloxycarbonyl. Preferred protecting groups are TMS, TES and TBS.

Suitable solvents include: N,N-dimethyl formamide (DMF), toluene, tetrahydrofuran, and dichloromethane, hexamethylphosphoramide (HMPA), 1-methyl-2-pyrrolidinone (NMP), 1-ethyl-2-pyrrolidinone (NEP), 1,3-dimethyl-3,4,5,6-tetrahydro-2(1H)-pyrimidinone (DMPU), 1,3-dimethyl-2-imidazolindinone. Preferable solvents are dichloromethane, HMPA, DMF, NMP, NEP, DMPU or a combination thereof.

Suitable catalysts include rhodium and palladium catalysts such as Pd(O) and Pd(II) complexes, such as PdCl₂, Pd(OAc)₂, Pd(dba)₂, Pd₂(dba)₃-CHCl₃, Rh(II) complexes, Rh(OAc)₂, Rh(CO₂C₁₋₈ alkyl), wherein "dba" represents dibenzylideneacetone. Preferably, the catalyst are Pd(dba)₂ or Pd(OAc)₂.

Suitable phosphine ligands include: 1,3-bis(diphenylphosphino)propane (DPPP); 1,2-bis(diphenylphosphino)-ethane (DPPE); 1,1'-bis(diphenylphosphino)ferrocene (DPPF); or PR^{a}R^{b}R^{c}, wherein R^{a},R^{b}, and R^{c} represent, independently: 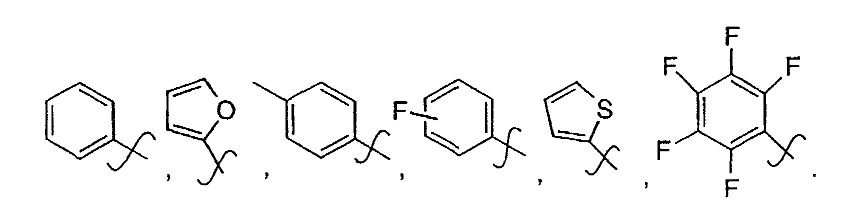 In a preferred aspect of the invention, the phosphine ligand employed is:

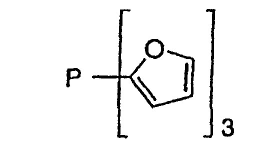

Suitable trifluoromethanesulfonylation reagents include: trifluoromethanesulfonic (triflic) anhydride (Tf20), trifluoromethanesulfonyl chloride (TfCl), and N-phenyltrifluoromethanesulfonimide. Suitable fluorosulfonylation reagents include S₂O₅F₂. Preferably, Tf₂O is the reagent employed.

Suitable amines include triethylamine (TEA), diisopropylethylamine (DIEPA), 2,2,6,6-tetramethylpiperidine, diethylamine, diisopropylamine and t-butylamine. Preferably, the amine chosen is DIEPA or 2,2,6,6-tetramethylpiperidine or a combination thereof.

In another preferred aspect of this invention a compound of formula I' which is useful in the synthesis of a variety of carbapenem antibiotics is described: 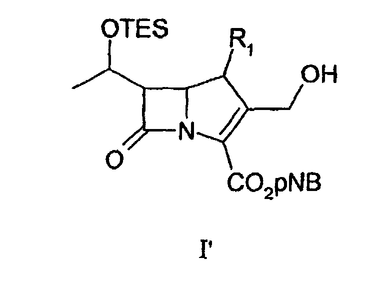 wherein R₁ represents CH₃ or H.

In another preferred aspect of this invention, the crystalline compound is:

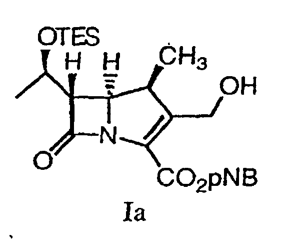

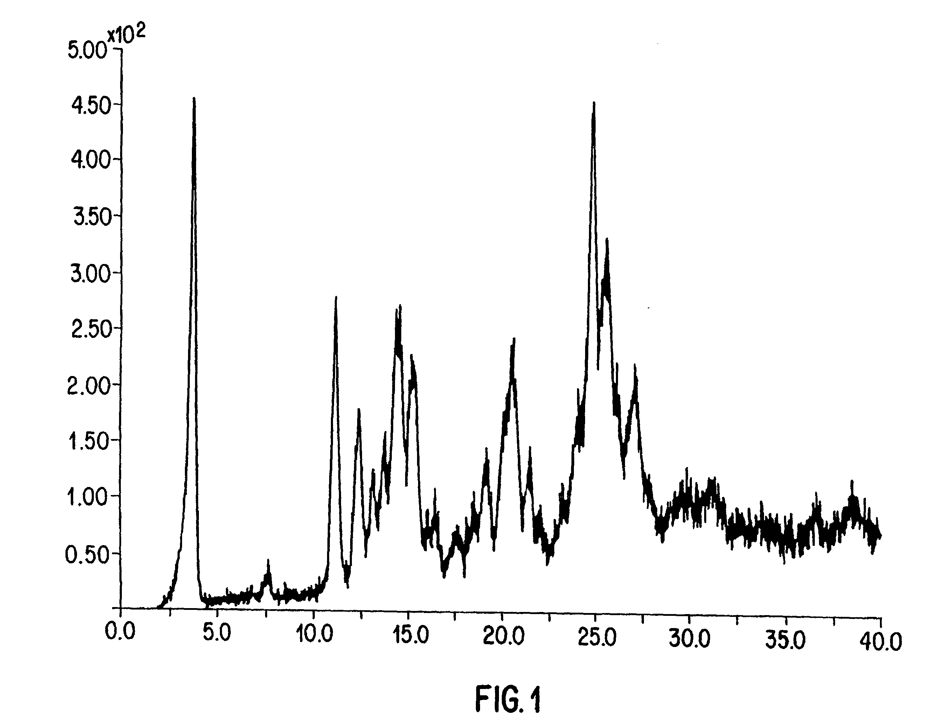

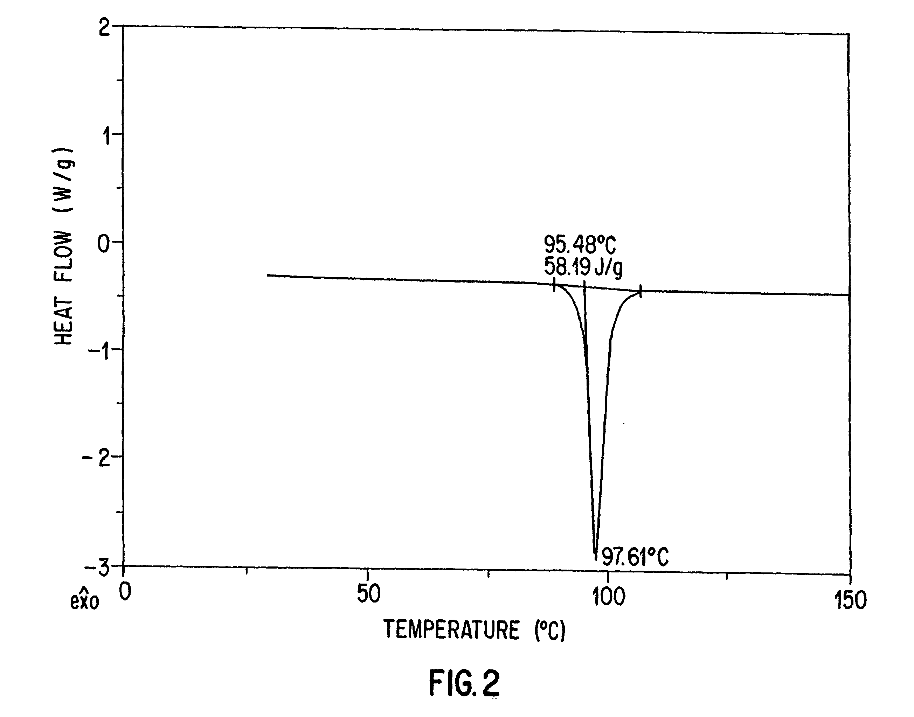

The compounds of the present invention may be isolated in various crystal forms, such as that illustrated by Figs. 1 and 2. This encompasses compounds of stable crystal structure, and is not limited to the crystal form described in the example.

The present invention is illustrated by the following non-limiting example.

### EXAMPLE

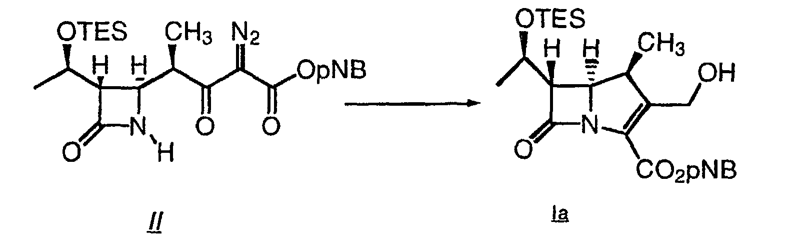

### Synthesis of Compound Ia

To a 72 L round bottom flask equipped with a mechanical stirrer, an argon inlet, a reflux condenser and a thermocouple was added compound II (2.7 Kg) and methylene chloride (13.5 L). Rhodium Octanonate (21.4 g) and zinc chloride (10.7 g) were added to the solution The solution was slowly heated to reflux (nitrogen is evolved during the course of reaction). The solution was refluxed for two hours and then cooled to room temperature. The HPLC assay indicated that the starting material had been consumed. The solution was stored overnight under argon.

The reaction mixture was cooled with a dry ice acetone bath to -50 °C. Tetramethylpiperidine (910 mL) and diisopropylethylamine (326 mL) were added over 15 minutes keeping temperature below -40 °C. Triflic anhydride (990 mL) was then added over 30 minutes keeping temperature below -40 °C. The resulting solution was aged at -50 °C for one hour. HMPA (5.35 L) was added. The solution was then warmed to 20 °C under vacuum for one hour to remove methylene chloride. Bu₃SnCH₂OH (88 wt % pure; 6 L) was added to this dark solution.

To a separate 72 L round bottom flask equipped with a mechanical stirrer, an argon inlet, and a thermocouple was added HMPA (16 L). Tri-2-furyl-phosphine (400 g), Pd(dba)₂ (400 g) and zinc chloride (730 g) was added to the HMPA and then heated to 70 °C. The enol triflate solution from above was transferred to the catalyst solution over 60 minutes at 70 °C. After the addition was completed the reaction was aged at 70 °C for one hour. All operations were carried out under argon.

The reaction mixture was cooled to room temperature. The reaction mixture was pumped into a extractor containing cold MTBE (20 L) and the reaction flask was rinsed with a additional MTBE (15 L). Cold water (35 L) was pumped into the extractor and the two phase solution was agitated for 10 minutes. The layers were allowed to separate and the organic layer was washed with cold water (35 L). The organic layer was separated and concentrated under vacuum. The concentrate was diluted with heptane and loaded on a silica gel column and eluted with EtOAc/Heptane. The fractions, containing the desired compound, were collected and concentrated, and the product crystallized during concentration. The solids were collected and dried (1.53 Kg; 79.5 wt %; 46.5 isolated yield).

Analytically pure material was obtained by silica gel column chromatography (EtOAc/Heptane), and subsequent crystallization from heptane. The melting point was measured using DSC thermography. The DSC instrument was a TA Instrument DSC 9210 and the DSC curve run at a heating rate of 10°C/min under a nitrogen flow of about 30 mL/min from room temperature to 250°C. A single endotherm (melting endotherm) was detected with a peak temperature of 98°C, an extrapolated onset temperature of 95°C and a heat of melting of 58 Joules/gm. (See Fig. 2).

NMR spectroscopy yielded the following results: ¹H NMR (CDCl₃, 250 MHz) δ 8.22 (m, 2H), 7.64 (d, J=8.7 Hz, 2H), 5.47 (AB d, J=13.9 Hz, 1H), 5.27 (AB d, J=13.9 Hz, 1H), 4.54 (AB dd, J=14.9, 6.6 Hz, 1H), 4.38 (AB dd, J=14.9, 5.9 Hz, 1H), 4.25 (m, 1H), 4.22 (dd, J=7.0, 3.0 Hz, 1H), 3.27 (dd, J=5.7, 3.0 Hz, 1H), 3.24 (m, 1H), 3.09 (m, 1H), 1.26 (d, J=6.2 Hz, 3H), 1.21 (d, J=7.4 Hz, 3H), 0.94 (t, J=7.9 Hz, 9H), 0.59 (m, 6H). ¹³C NMR (CDCl₃, 63 MHz) δ 175.1, 161.8, 153.5, 147.6, 142.4, 128.0, 127.3, 123.7, 65.8, 65.6, 60.3, 57.6, 55.9, 41.6, 22.5, 15.3, 6.7, 4.9.

The X-ray crystallography was conducted using a Phillips APD3720 using CuKa radiation. The powder pattern was run at 0.075 2-θ/sec from about 2 to about 40 2-θ. The x-ray crystallography pattern yielded the results summarized in Fig.1 and Table 1, below.

**TABLE 1**

| Peak Angle (°) | d-spacing (Å) | Relative Intensity (%) |
|---|---|---|
| 3.8 | 23.3 | 100 |
| 7.7 | 11.5 | 6 |
| 11.3 | 7.85 | 56 |
| 12.5 | 7.08 | 38 |
| 13.2 | 6.69 | 22 |
| 13.8 | 6.41 | 28 |
| 14.5 | 6.11 | 57 |
| 18.5 | 4.79 | 11 |
| 19.1 | 4.64 | 18 |
| 20.6 | 4.31 | 43 |
| 21.5 | 4.12 | 19 |
| 24.9 | 3.57 | 89 |
| 25.6 | 3.84 | 56 |
| 27.1 | 3.29 | 30 |
| 29.5 | 3.02 | 6 |
| 31.2 | 2.87 | 9 |
| 36.5 | 2.46 | 6 |
| 38.4 | 2.34 | 7 |

## Claims

1. A process for preparation of a compound of formula I: 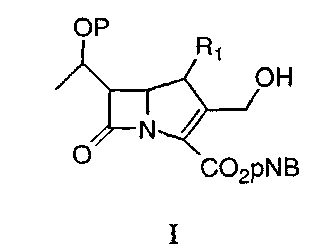 wherein:
R₁ represents CH3 or H; and P represents a protecting group;
comprising reacting a compound of formula IV: 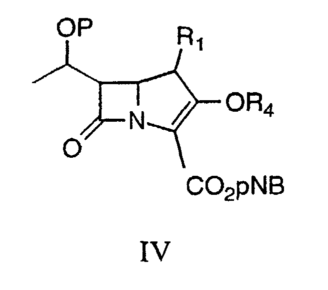 wherein R₁ and P and are defined above and R4 represents triflate or SO₂F;
in the presence of a catalyst and (R₃)₃SnCH₂OP", wherein : each R3 represents C₁₋₄ lower alkyl, and P" represents H or a protecting group, to yield the compound of formula I.

2. A process according to claim 1 further comprising optionally adding a Lewis acid selected from the group consisting of ZnBr₂, ZnCl₂, MgCl₂, MgBr₂, CaCl₂ and CaBr₂.

3. A process according to claim 2 wherein the Lewis acid is ZnCl₂.

4. A process according to claim 1 further comprising adding a phosphine ligand selected from the group consisting of: 1,3-bis(diphenylphosphino)-propane (DPPP); 1,2-bis(diphenylphosphino)-ethane (DPPE); 1,1'-bis-(diphenylphosphino)ferrocene (DPPF); and PR^{a}R^{b}R^{c}, wherein R^{a},R^{b}, and R^{c} represent, independently:

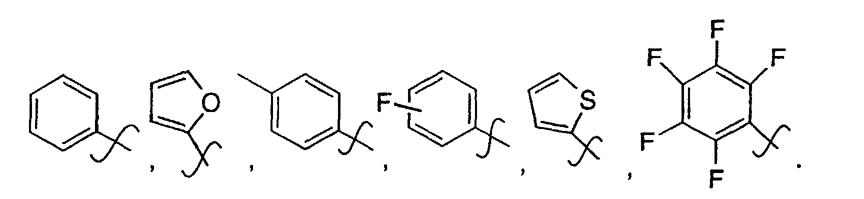

5. A process in accordance with claim 4 wherein the phosphine ligand is:

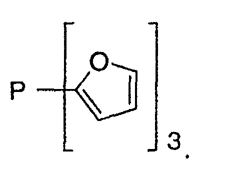

6. A process according to claim 1 wherein the catalyst is selected from the group consisting of Pd(II) complexes, PdCl₂, Pd(OAc)₂, Pd(dba)₂, Pd₂(dba)₃-CHCl₃, Rh(II) complexes, Rh(OAc)₂, Rh(CO₂C₁₋₈ alkyl) and Rh(OCOC₁₋₈ alkyl).

7. A process in accordance with claim 5 wherein the Palladium catalyst are Pd(dba)₂ or Pd(OAc)₂.
and the phosphine ligand is:

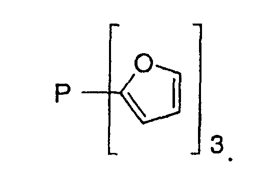

8. A process in accordance with claim 1 wherein the protecting group P is selected from the group consisting of t-butylmethylphenylsilyl, t-butyldiphenylsilyl, trimethylsilyl (TMS), triethylsilyl (TES), t-butyldimethylsilyl (TBS), o-nitrobenzyloxycarbonyl, p-nitrobenzyloxycarbonyl (pNZ), p-nitrobenzyl (pNB), benzyloxycarbonyl, t-butyloxycarbonyl, 2,2,2-trichloroethyloxycarbonyl and allyloxycarbonyl.

9. A process in accordance with claim 8 wherein the protecting group is TMS, TES or TBS.

10. A process according to claim 1 wherein R₁ is methyl.

11. A process according to claim 1 which is carried out under inert conditions.

12. A process according to claim 11 wherein the inert condition comprises using nitrogen or argon.

13. A process for preparation of a compound of formula I: 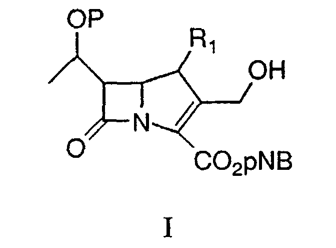 wherein:
wherein R₁ represents CH₃ and P represents TES, TMS or TBS; comprising:
reacting a compound of formula IV: 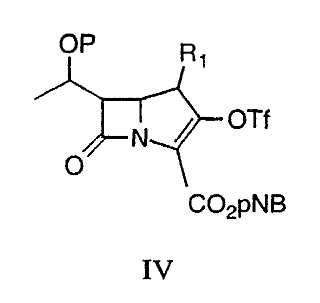 wherein P and R₁ are defined above;
in the presence of ZnCl₂, Pd(dba)₂, 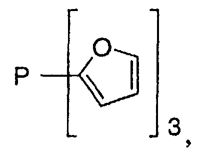 and
(R₃)₃SnCH₂OP", wherein:
R3 represents a C₁₋₄ lower alkyl, and P" represents H, TES, TMS or TBS; to yield the compound of formula I, said process conducted under inert conditions using nitrogen or argon.

14. A process for preparation of a compound of formula I: 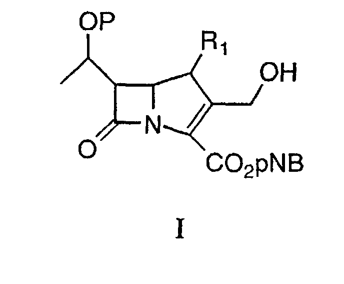 wherein:
R₁ represents CH3 or H;
and P represents a protecting group; comprising:
step (1) heating a compound of formula II: 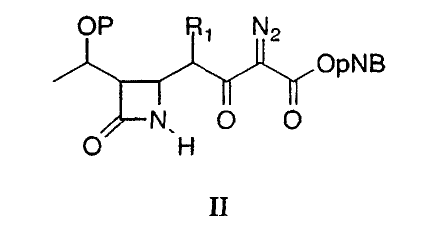 wherein R₁ and P are defined above;
in the presence of 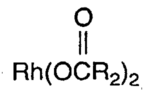 wherein R₂ is C₁₋₁₅ alkyl, to yield a compound of formula III: 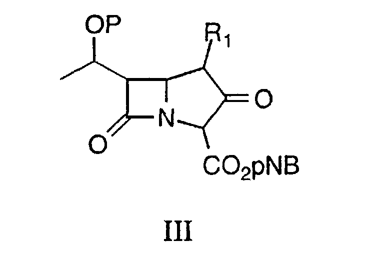 wherein R₁ and P are defined above;
step (2) reacting a compound of formula III in the presence of an amine, or combination of amines, and a trifluoromethanesulfonylation reagent or fluorosulfonylation reagent, to yield a compound of formula IV: 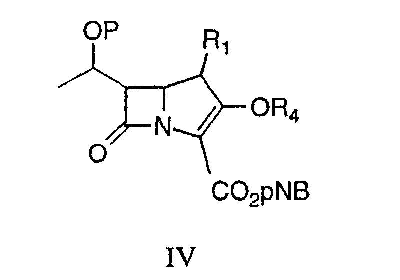 wherein R₁ and P and are defined above and R4 represents triflate or SO₂F;
step (3) reacting compound IV in the presence of a catalyst, and (R3)3SnCH20P", wherein R3 represents a C₁₋₄ lower alkyl, and P" represents H or a protecting group, to yield the compound of formula I.

15. A process according to claim 14 further comprising optionally adding a Lewis acid, selected from the group consisting of ZnBr₂, ZnCl₂, MgCl₂, MgBr₂, CaCl₂, and CaBr₂ to the first and/or third steps.

16. A process according to claim 15 wherein the Lewis acid added to both steps is ZnCl₂.

17. A process according to claim 14 comprising further adding a phosphine ligand, selected from the group consisting of 1,3-bis-(diphenylphosphino)propane (DPPP); 1,2-ethyl(diphenylphosphino)-ethane (DPPE); 1,1'-bis(diphenylphosphino)ferrocene (DPPF); and PR^{a}R^{b}R^{c}, wherein R^{a},R^{b}, and R^{c} represent, independently:

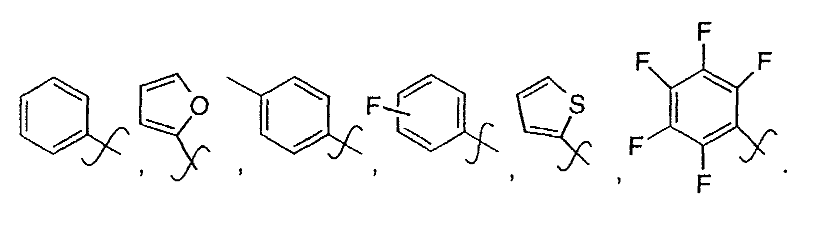

18. A process in accordance with claim 17 wherein the phosphine ligand is:

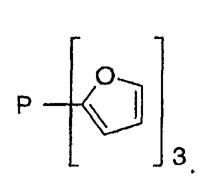

19. A process according to claim 14 wherein catalyst is selected from the group consisting of of Pd(II) complexes, PdCl₂, Pd(OAc)₂, Pd(dba)₂, Pd₂(dba)₃-CHCl₃, Rh(II) complexes, Rh(OAc)₂, and Rh(CO₂C₁₋₈ alkyl..

20. A process in accordance with claim 19 wherein the Palladium catalyst is Pd(dba)₂.

21. A process in accordance with claim 14 wherein the protecting group P is selected from the group consisting of t-butylmethylphenylsilyl, t-butyldiphenylsilyl, trimethylsilyl (TMS), triethylsilyl (TES), t-butyldimethylsilyl (TBS), o-nitrobenzyloxycarbonyl, p-nitrobenzyloxycarbonyl (PNZ), p-nitrobenzyl (PNB), benzyloxycarbonyl, t-butyloxycarbonyl, 2,2,2-trichloroethyloxycarbonyl and allyloxycarbonyl.

22. A process in accordance with claim 21 wherein the protecting group is TMS, TES or TBS.

23. A process in accordance with claim 14 wherein R₁ is methyl and R₂ is C₁₋₁₂ alkyl.

24. A process in accordance with claim 14 wherein the trifluoromethanesulfonylation reagent is selected from the group consisting of Tf₂O, TfCl, and N-phenyltrifluoromethanesulfonimide and the fluorosulfonylation agent is S2O₅F₂.

25. A process in accordance with claim 24 wherein the trifluoromethanesulfonylation reagent used is Tf₂O.

26. A process in accordance with claim 14 wherein the amine reagent is selected from the group consisting of triethylamine, diisopropylethylamine, 2,2,6,6-tetramethylpiperidine, diisopropylamine, t-butylamine and diethylamine.

27. A process in accordance with claim 26 wherein the amine employed is DIEPA or 2,2,6,6-tetramethylpiperidine, or a combination thereof.

28. A process according to claim 14 which is carried out under inert conditions.

29. A process according to claim 28 wherein the inert condition comprises using nitrogen or argon.

30. A process for preparation of a compound of formula I: 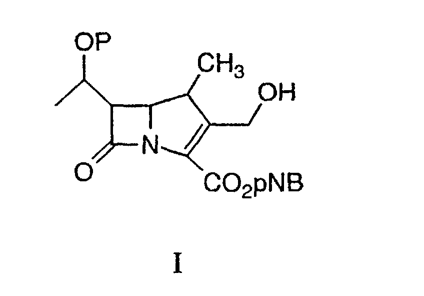 wherein P represents TES, TMS or TBS;
comprising refluxing a compound of formula II: 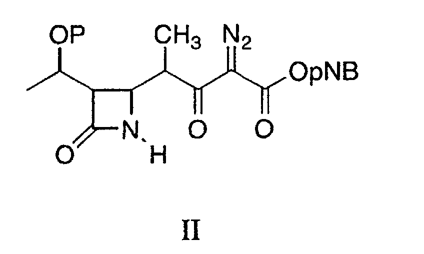 wherein P is defined above;
in the presence of ZnCl2 and 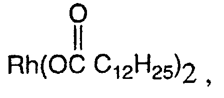 to yield a compound of formula III: 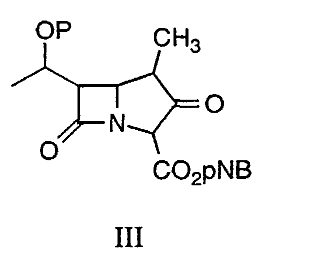 wherein P is defined above;
reacting a compound of formula III in the presence of DIEPA or 2,2,6,6-tetramethylpiperidine, or a combination thereof, and Tf₂O, to yield a compound of formula IV: 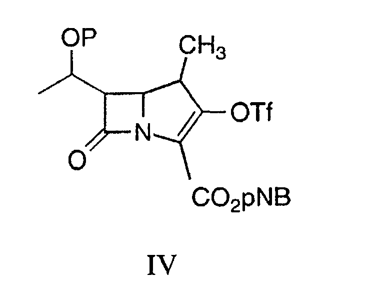 wherein P and is defined above;
reacting compound IV in the presence of a ZnCl₂, Pd(dba)₂, 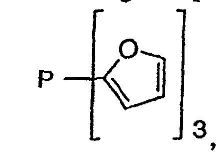 and (R₃)₃SnCH₂OP", wherein R3 represents a C₁₋₄ lower alkyl, and P" represents H, TMS, TES, or TBS; to yield the compound of formula I, said process conducted under inert conditions using nitrogen or argon.

31. A compound of formula I: 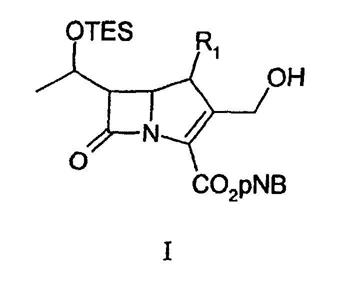 wherein R₁ represents CH₃ or H.

32. A crystalline compound of formula Ia:

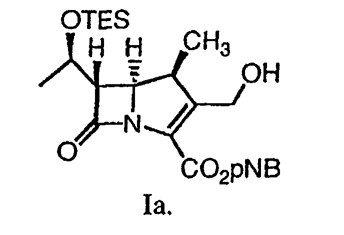

33. A crystalline compound of formula Ia in accordance with claim 32 having an X-ray crystallography pattern as shown in Figure 1.

34. A crystalline compound of formula Ia in accordance with claim 32 having a DSC thermogram as shown in Figure 2.

## Patentansprüche

1. Ein Verfahren zur Herstellung einer Verbindung der Formel I: 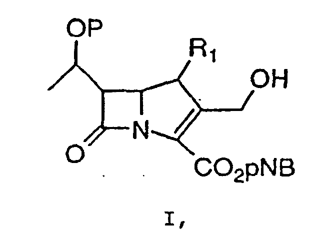 wobei:
R₁ CH₃ oder H bedeutet und P eine Schutzgruppe bedeutet,
umfassend die Umsetzung einer Verbindung der Formel IV: 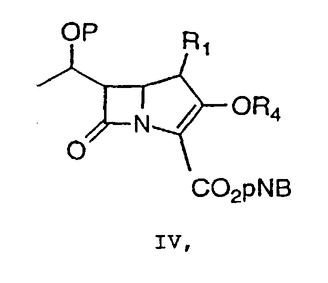 wobei R₁ und P oben definiert sind und R₄ Triflat oder SO₂F bedeutet,
in Gegenwart eines Katalysators und (R₃)₃SnCH₂OP", wobei: jedes R₃ C₁₋₄-Niedrigalkyl bedeutet und P" H oder eine Schutzgruppe bedeutet, um die Verbindung der Formel I zu ergeben.

2. Ein Verfahren gemäß Anspruch 1, das ferner gegebenenfalls die Zugabe einer Lewissäure, ausgewählt aus der Gruppe, bestehend aus ZnBr₂, ZnCl₂, MgCl₂, MgBr₂, CaCl₂ und CaBr₂, umfaßt.

3. Ein Verfahren gemäß Anspruch 2, wobei die Lewissäure ZnCl₂ ist.

4. Ein Verfahren gemäß Anspruch 1, das ferner die Zugabe eines Phosphinliganden, ausgewählt aus der Gruppe, bestehend aus: 1,3-Bis(diphenylphosphino)propan (DPPP), 1,2-Bis(diphenylphosphino)ethan (DPPE), 1,1'-Bis(diphenylphosphino)ferrocen (DPPF) und PR^{a}R^{b}R^{c}, wobei R^{a}, R^{b} und R^{c} unabhängig bedeuten: 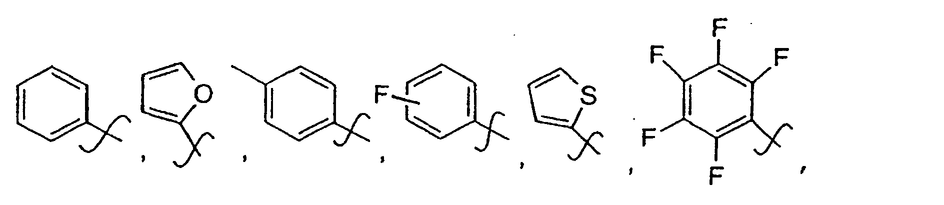 umfaßt.

5. Ein Verfahren gemäß Anspruch 4, wobei der Phosphinligand ist:

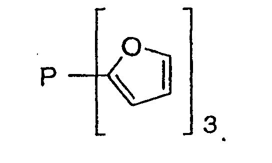

6. Ein Verfahren gemäß Anspruch 1, wobei der Katalysator ausgewählt ist aus der Gruppe, bestehend aus Pd(II)-Komplexen, PdCl₂, Pd(OAc)₂, Pd(dba)₂, pd₂(dba)₃-CHCl₃, Rh(II)-Komplexen, Rh(OAc)₂, Rh(CO₂C₁₋₈-Alkyl) und Rh (OCOC₁₋₈-Alkyl).

7. Ein Verfahren gemäß Anspruch 5, wobei der Palladiumkatalysator Pd(dba)₂ oder Pd(OAc)₂ ist und der Phosphinligand ist:

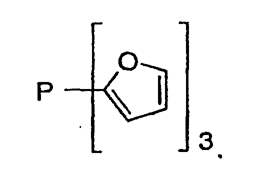

8. Ein Verfahren gemäß Anspruch 1, wobei die Schutzgruppe P ausgewählt ist aus der Gruppe, bestehend aus t-Butylmethylphenylsilyl, t-Butyldiphenylsilyl, Trimethylsilyl (TMS), Triethylsilyl (TES), t-Butyldimethylsilyl (TBS), o-Nitrobenzyloxycarbonyl, p-Nitrobenzyloxycarbonyl (pNZ), p-Nitrobenzyl (pNB), Benzyloxycarbonyl, t-Butyloxycarbonyl, 2,2,2-Trichlorethyloxycarbonyl und Allyloxycarbonyl.

9. Ein Verfahren gemäß Anspruch 8, wobei die Schutzgruppe TMS, TES oder TBS ist.

10. Ein Verfahren gemäß Anspruch 1, wobei R₁ Methyl ist.

11. Ein Verfahren gemäß Anspruch 1 das unter inerten Bedingungen durchgeführt wird.

12. Ein Verfahren gemäß Anspruch 11, wobei die inerte Bedingung die Verwendung von Stickstoff oder Argon umfaßt.

13. Ein Verfahren zur Herstellung einer Verbindung der Formel I: 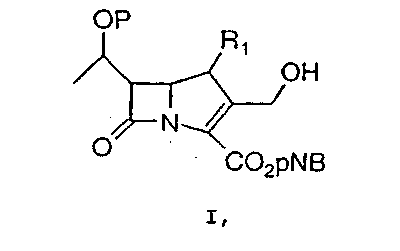 wobei:
R₁ CH₃ bedeutet und P TES, TMS oder TBS bedeutet,
umfassend:
Umsetzung einer Verbindung der Formel IV: 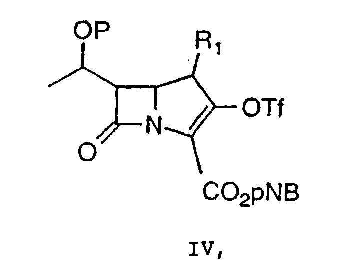 wobei P und R₁ oben definiert sind,
in Gegenwart von ZnCl₂, Pd(dba)₂, 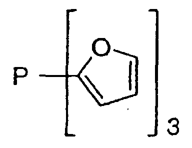 und
(R₃)₃SnCH₂OP", wobei:
R₃ ein C₁₋₄-Niedrigalkyl bedeutet und P" H, TES, TMS oder TBS bedeutet, um die Verbindung der Formel I zu ergeben, wobei das Verfahren unter inerten Bedingungen unter Verwendung von Stickstoff oder Argon durchgeführt wird.

14. Ein Verfahren zur Herstellung einer Verbindung der Formel I: 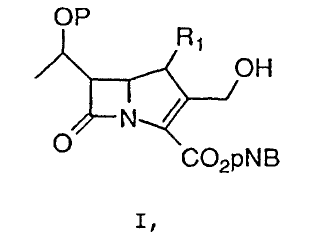 wobei:
R₁ CH₃ oder H bedeutet
und P eine Schutzgruppe bedeutet, umfassend:
Schritt (1) Erwärmen einer Verbindung der Formel II: 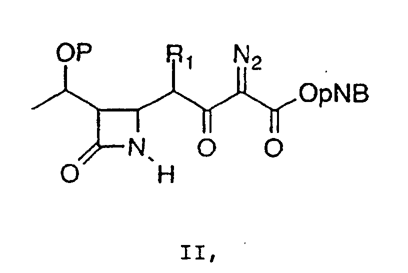 wobei R₁ und P oben definiert sind,
in Gegenwart von 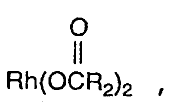 wobei R₂ C₁₋₁₅-Alkyl ist, um eine Verbindung der Formel III zu ergeben: 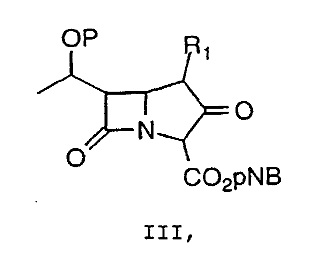 wobei R₁ und P oben definiert sind,
Schritt (2) Umsetzen einer Verbindung der Formel III in Gegenwart eines Amins oder einer Kombination aus Amihen und eines Trifluormethansulfonylierungsreagenzes oder Fluorsulfonylierungsreagenzes, um eine Verbindung der Formel IV zu ergeben: 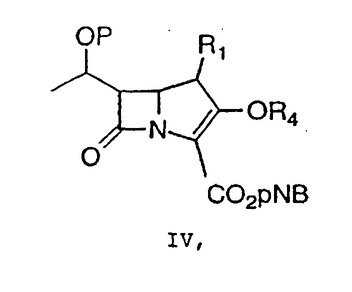 wobei R₁ und P oben definiert sind und R₄ Triflat oder SO₂F bedeutet,
Schritt (3) Umsetzen von Verbindung IV in Gegenwart eines Katalysators und (R₃)₃SnCH₂OP", wobei R₃ ein C₁₋₄-Niedrigalkyl bedeutet und P" H oder eine Schutzgruppe bedeutet, um die Verbindung der Formel I zu ergeben.

15. Ein Verfahren gemäß Anspruch 14, das ferner gegebenenfalls die Zugabe einer Lewissäure, ausgewählt aus der Gruppe, bestehend aus ZnBr₂, ZnCl₂, MgCl₂, MgBr₂, CaCl₂ und CaBr₂, zum ersten und/oder dritten Schritt umfaßt.

16. Ein Verfahren gemäß Anspruch 15, wobei die zu beiden Schritten hinzugegebene Lewissäure ZnCl₂ ist.

17. Ein Verfahren gemäß Anspruch 14, das ferner die Zugabe eines Phosphinliganden, ausgewählt aus der Gruppe, bestehend aus 1,3-Bis(diphenylphosphino)propan (DPPP), 1,2-Bis(diphenylphosphino)ethan (DPPE), 1,1'-Bis(diphenylphosphino)ferrocen (DPPF) und PR^{a}R^{b}R^{c}, wobei R^{a}, R^{b} und R^{c} unabhängig bedeuten: 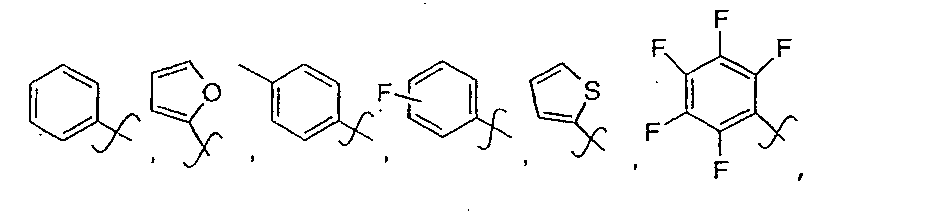 umfaßt.

18. Ein Verfahren gemäß Anspruch 17, wobei der Phosphinligand ist:

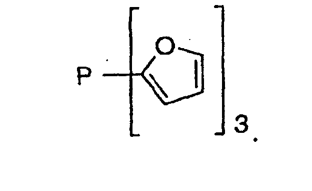

19. Ein Verfahren gemäß Anspruch 14, wobei der Katalysator ausgewählt ist aus der Gruppe, bestehend aus Pd(II)-Komplexen, PdCl₂, Pd(OAc)₂, Pd(dba)₂, Pd₂(dba)₃-CHCl₃, Rh(II)-Komplexen, Rh(OAc)₂ und Rh(CO₂C₁₋₈-Alkyl).

20. Ein Verfahren gemäß Anspruch 19, wobei der Palladiumkatalysator Pd(dba)₂ ist.

21. Ein Verfahren gemäß Anspruch 14, wobei die Schutzgruppe P ausgewählt ist aus der Gruppe, bestehend aus t-Butylmethylphenylsilyl, t-Butyldiphenylsilyl, Trimethylsilyl (TMS), Triethylsilyl (TES), t-Butyldimethylsilyl (TBS), o-Nitrobenzyloxycarbonyl, p-Nitrobenzyloxycarbonyl (PNZ), p-Nitrobenzyl (PNB), Benzyloxycarbonyl, t-Butyloxycarbonyl, 2,2,2-Trichlorethyloxycarbonyl und Allyloxycarbonyl.

22. Ein Verfahren gemäß Anspruch 21, wobei die Schutzgruppe TMS, TES oder TBS ist.

23. Ein Verfahren gemäß Anspruch 14, wobei R₁ Methyl ist und R₂ C₁₋₁₂-Alkyl ist.

24. Ein Verfahren gemäß Anspruch 14, wobei das Trifluormethansulfonylierungsreagenz ausgewählt ist aus der Gruppe, bestehend aus Tf₂O, TfCl und N-Phenyltrifluormethansulfonimid, und das Fluorsulfonylierungsreagenz S₂O₅F₂ ist.

25. Ein Verfahren gemäß Anspruch 24, wobei das verwendete Trifluormethansulfonylierungsreagenz Tf₂O ist.

26. Ein Verfahren gemäß Anspruch 14, wobei das Aminreagenz ausgewählt ist aus der Gruppe, bestehend aus Triethylamin, Diisopropylethylamin, 2,2,6,6-Tetramethylpiperidin, Diisopropylamin, t-Butylamin und Diethylamin.

27. Ein Verfahren gemäß Anspruch 26, wobei das eingesetzte Amin DIEPA oder 2,2,6,6-Tetramethylpiperidin oder eine Kombination davon ist.

28. Ein Verfahren gemäß Anspruch 14, das unter inerten Bedingungen durchgeführt wird.

29. Ein Verfahren gemäß Anspruch 28, wobei die inerte Bedingung die Verwendung von Stickstoff oder Argon umfaßt.

30. Ein Verfahren zur Herstellung einer Verbindung der Formel I: 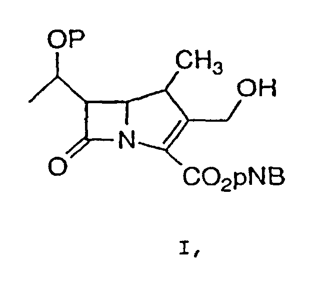 wobei P TES, TMS oder TBS bedeutet,
umfassend das Refluxieren einer Verbindung der Formel II: 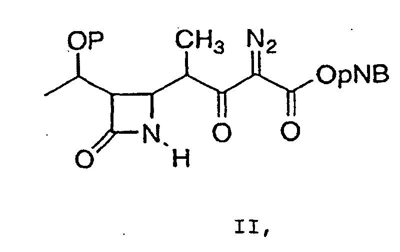 wobei P oben definiert ist,
in Gegenwart von ZnCl₂ und 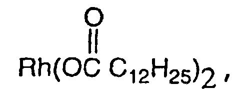 um eine Verbindung der Formel III zu ergeben: 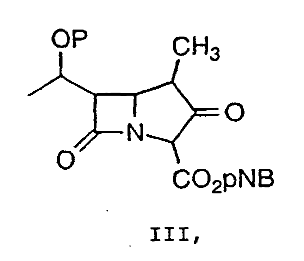 wobei P oben definiert ist,
das Umsetzen einer Verbindung der Formel III in Gegenwart von DIEPA oder 2,2,6,6-Tetramethylpiperidin oder einer Kombination davon und Tf₂O, um eine Verbindung der Formel IV zu ergeben: 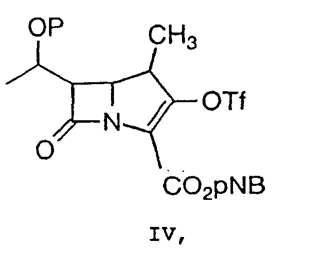 wobei P oben definiert ist,
das Umsetzen von Verbindung IV in Gegenwart von ZnCl₂, Pd(dba)₂, 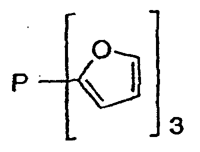 und (R₃)₃SnCH₂OP", wobei R₃ ein C₁₋₄-Niedrigalkyl bedeutet und P" H, TMS, TES oder TBS bedeutet, um die Verbindung der Formel I zu ergeben, wobei das Verfahren unter inerten Bedingungen unter Verwendung von Stickstoff oder Argon durchgeführt wird.

31. Eine Verbindung der Formel I: 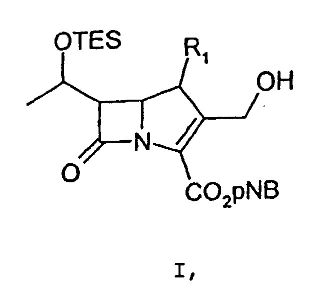 wobei R₁ CH₃ oder H bedeutet.

32. Eine kristalline Verbindung der Formel Ia:

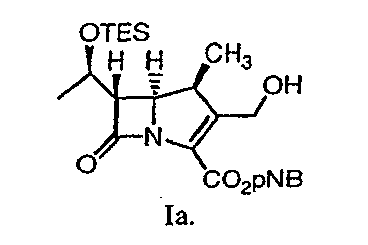

33. Eine kristalline Verbindung der Formel Ia gemäß Anspruch 32 mit einem Röntgenkristallographiemuster, wie es in Figur 1 gezeigt ist.

34. Eine kristalline Verbindung der Formel Ia gemäß Anspruch 32 mit einem DSC-Thermogramm, wie es in Figur 2 gezeigt ist.

## Revendications

1. Procédé de préparation d'un composé de formule I dans laquelle: 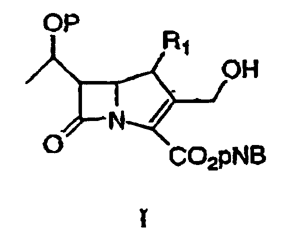 R₁ représente CH₃ ou H; et P représente un groupe protecteur;
comprenant le fait de faire réagir un composé de formule IV: 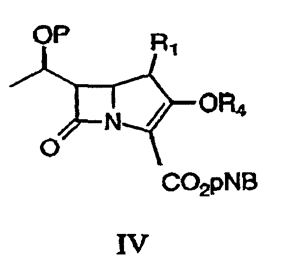 dans laquelle R₁ et P sont définis ci-dessus et R₄ représente un groupe triflate ou SO₂F;
en présence d'un catalyseur, et (R₃)₃SnCH₂OP", où chaque radical R₃ représente un groupe alkyle inférieur en C₁₋₄, et P" représente H ou un groupe protecteur, pour produire le composé de formule I.

2. Procédé selon la revendication 1 comprenant en outre le fait d'ajouter éventuellement un acide de Lewis choisi dans le groupe constitué par ZnBr₂, ZnCl₂, MgCl₂, MgBr₂, CaCl₂ et CaBr₂.

3. Procédé selon la revendication 2 dans lequel l'acide de Lewis est ZnCl₂.

4. Procédé selon la revendication 1 comprenant en outre le fait d'ajouter un ligand phosphine choisi dans le groupe constitué par: le 1,3-bis(diphénylphosphino)propanc (DPPP); le 1,2-bis(diphénylphosphino)-éthane (DPPE); le 1,1'-bis-(diphénylphosphino)ferrocène (DPPF); et PR^{a}R^{b}R^{c}, où R^{a}, R^{b} et R^{c} représentent, indépendamment les uns des autres:

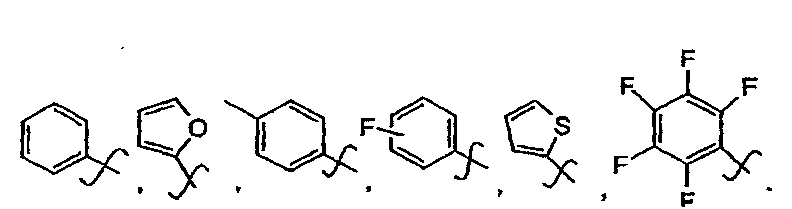

5. Procédé selon la revendication 4 dans lequel le ligand phosphine est:

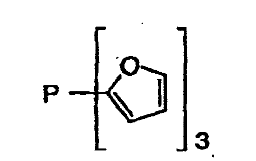

6. Procédé selon la revendication 1 dans lequel le catalyseur est choisi dans le groupe constitué par les complexes de Pd(II), PdCl₂, Pd(OAc)₂, Pd(dba)₂, Pd₂(dba)₃-CHCl₃, les complexes de Rh(II), Rh(OAc)₂, Rh(CO₂-alkyle en C₁₋₈) et Rh(OCO-alkyle en C₁₋₈).

7. Procédé selon la revendication 5 dans lequel le catalyseur au palladium est Pd(dba)₂ ou Pd(OAc)₂
et le ligand phosphine est:

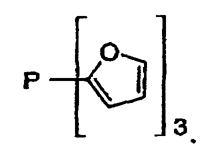

8. Procédé selon la revendication 1 dans lequel le groupe protecteur P est choisi dans le groupe constitué par les groupes t-butylméthylphénylsilyle, t-butyldiphénylsilyle, triméthylsilyle (TMS), triéthylsilyle (TES), t-butyldiméthylsilyle (TBS), o-nitrobenzyloxycarbonyle, p-nitrobenzyloxycarbonyle (pNZ), p-nitrobenzyle (pNB), benzyloxycarbonyle, t-butyloxycarbonyle, 2,2,2-trichloroéthyloxycarbonyle et allyloxycarbonyle.

9. Procédé selon la revendication 8 dans lequel le groupe protecteur est TMS, TES ou TBS.

10. Procédé selon la revendication 1 dans lequel R₁ est un groupe méthyle.

11. Procédé selon la revendication 1 qui est mis en oeuvre dans des conditions inertes.

12. Procédé selon la revendication 11 dans lequel les conditions inertes font appel à l'emploi d'azote ou d'argon.

13. Procédé de préparation d'un composé de formule I: 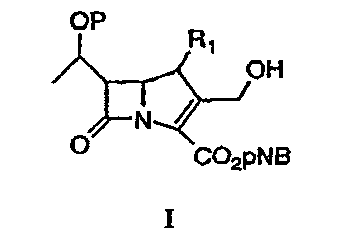 dans laquelle R₁ représente CH₃ et P représente TES, TMS ou TBS;
comprenant :
le fait de faire réagir un composé de formule IV: 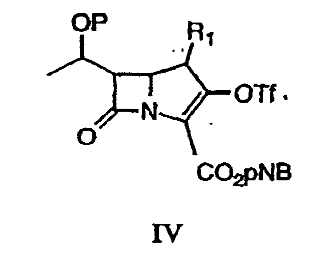 dans laquelle P et R₁ sont définis ci-dessus;
en présence de ZnCl₂, Pd(dba)₂, 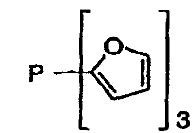 et
(R₃)₃SnCH₂OP", où :
R₃ représente un groupe alkyle inférieur en C₁₋₄, et P" représente H, TES, TMS ou TBS; pour produire le composé de formule I, ledit procédé étant mis en oeuvre dans des conditions inertes faisant appel à de l'azote ou de l'argon.

14. Procédé de préparation d'un composé de formule I: 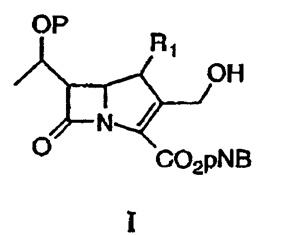 dans laquelle:
R₁ représente CH₃ ou H;
et P représente un groupe protecteur; comprenant :
l'étape (1) qui consiste à chauffer un composé de formule II: 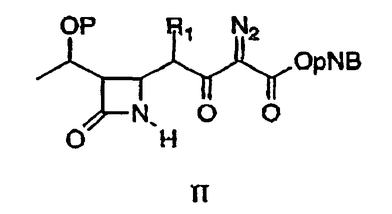 dans laquelle R₁ et P sont définis ci-dessus;
en présence de 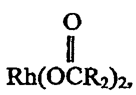 où R₂ est un groupe alkyle en C₁₋₁₅,
pour produire un composé de formule III: 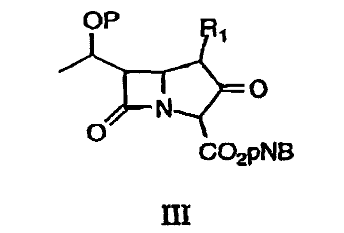 dans laquelle R₁ et P sont définis ci-dessus;
l'étape (2) qui consiste à faire réagir un composé de formule III en présence d'une amine, ou d'une combinaison d'amines, et un réactif de trifluorométhancsulfonylation ou un réactif de fluorosulfonylation, pour produire un composé de formule IV: 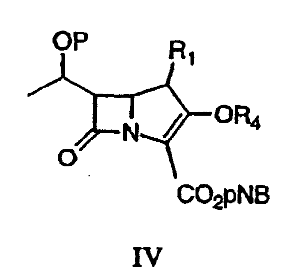 dans laquelle R₁ et P sont définis ci-dessus et R₄ représente un groupe triflate ou SO₂F;
l'étape (3) qui consiste à faire réagir un composé IV, en présence d'un catalyseur, et (R₃)₃SnCH₂OP", où R₃ représente un groupe alkyle inférieur en C₁₋₄, et P" représente H ou un groupe protecteur, pour produire le composé de formule I.

15. Procédé selon la revendication 14 comprenant en outre le fait d'ajouter éventuellement un acide de Lewis, choisi dans le groupe constitué par ZnBr₂, ZnCl₂, MgCl₂, MgBr₂, CaCl₂ et CaBr₂, à la première et/ou à la troisème étape.

16. Procédé selon la revendication 15 dans lequel l'acide de Lewis ajouté aux deux étapes est ZnCl₂.

17. Procédé selon la revendication 14 comprenant en outre le fait d'ajouter un ligand phosphine, choisi dans le groupe constitué par le 1,3-bis(diphénylphosphino)propane (DPPP); le I,2-éthyl(diphénylphosphino)-éthane (DPPE); le 1,1'-bis(diphénylphosphino)ferrocène (DPPF); et PR^{a}R^{b}R^{c}, où R^{a}, R^{b} et R^{c} représentent, indépendamment les uns des autres:

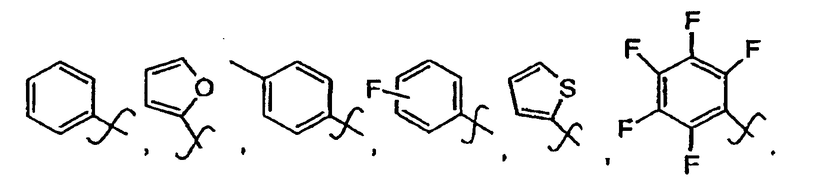

18. Procédé selon la revendication 17 dans lequel le ligand phosphine est:

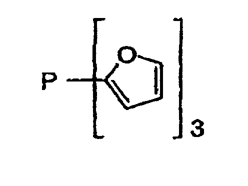

19. Procédé selon la revendication 14 dans lequel le catalyseur est choisi dans le groupe constitué par les complexes de Pd(II), PdCl₂, Pd(OAc)₂, Pd(dba)₂, Pd₂(dba)₃-CHCl₃, les complexes de Rh(H), Rh(OAc)₂, et Rh(CO₂-alkyle en C₁₋₈).

20. Procédé selon la revendication 19 dans lequel le catalyseur au palladium est Pd(dba)₂.

21. Procédé selon la revendication 14 dans lequel le groupe protecteur P est choisi dans le groupe constitué par les groupes t-butylméthylphénylsilyle, t-butyldiphénylsilyle, trriméthylsilyle (TMS), triéthylsilyle (TES), t-butyldiméthylsilyle (TBS), o-nitrobenzyloxycarbonyle, p-nitrobenzyloxycarbonyle (PNZ), p-nitrobenzyle (PNB), benzyloxycarbonyle, t-butyloxycarbonyle, 2,2,2-trichloroéthyloxycarbonyle et allyloxycarbonyle.

22. Procédé selon la revendication 21 dans lequel le groupe protecteur est TMS, TES ou TBS.

23. Procédé selon la revendication 14 dans lequel R₁ est un groupe méthyle et R₂ est un groupe alkyle en C₁₋₁₂.

24. Procédé selon la revendication 14 dans lequel le réactif de trifluorométhanesulfonylation est choisi dans le groupe constitué par Tf₂O, TfCl et le N-phényltrifluorométhanesulfonimide et l'agent de fluorosulfonylation est S₂O₅F₂.

25. Procédé selon la revendication 24 dans lequel le réactif de trifluorométhanesulfonylation utilisé est Tf₂O.

26. Procédé selon la revendication 14 dans lequel le réactif amine est choisi dans le groupe constitué par la triéthylamine, la diisopropyléthylamine, la 2,2,6,6-tétraméthylpipéridine, la diisopropylamine, la t-butylamine et la diéthylamine.

27. Procédé selon la revendication 26 dans lequel l'amine employée est la DIEPA ou la 2,2,6,6-tétraméthylpipéridine, ou une combinaison de celles-ci.

28. Procédé selon la revendication 14 qui est mis en oeuvre dans des conditions inertes.

29. Procédé selon la revendication 28 dans lequel les conditions inertes comprennent le fait de faire appel à de l'azote ou de l'argon.

30. Procédé de préparation d'un composé de formule I: 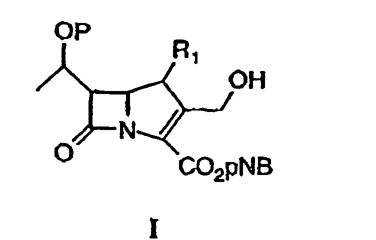 dans laquelle P représente TES, TMS ou TBS;
comprenant le fait de porter au reflux un composé de formule II: 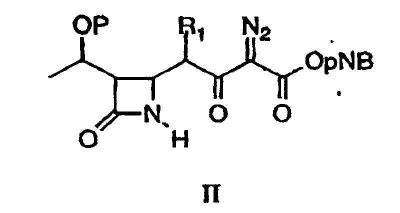 dans laquelle P est défini ci-dessus;
en présence de ZnCl₂ et de 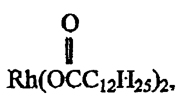 pour produire un composé de formule III: 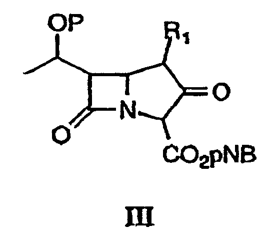 dans laquelle P est défini ci-dessus;
le fait de faire réagir un composé de formule III en présence de DIEPA ou de 2,2,6,6-tétraméthylpipéridine, ou d'une combinaison des deux, et Tf₂O, pour produire un c 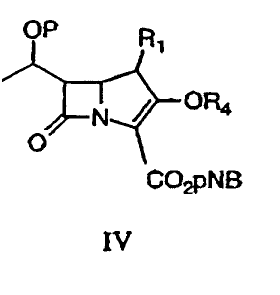 dans laquelle P et est défini ci-dessus;
le fait de faire réagir le composé IV en présencc de ZnC₂, de Pd(dba)₂, de 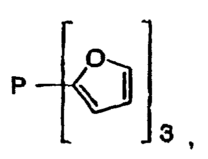 et (R₃)₃SnCH₂OP", où R₃ représente un groupe alkyle inférieur en C₁₋₄, et P" représente H, TMS, TES ou TBS; pour produire le composé de formule I, ledit procédé étant mis en oeuvre dans des conditions inertes faisant appel à de l'azote ou de l'argon.

31. Composé de formule I: 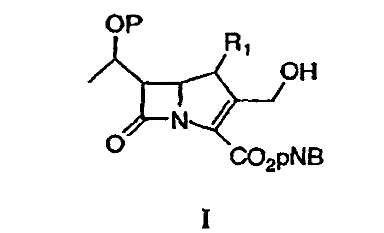 dans laquelle R₁ représente CH₃ ou H.

32. Composé cristallin de formule Ia:

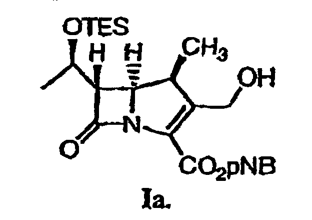

33. Composé cristallin de formule Ia selon la revendication 32 ayant un spectre en cristallographie aux rayons X tel que présenté sur la figure 1.

34. Composé cristallin de formule Ia selon la revendication 32 ayant une courbe d'analyse thermique par DSC telle que présentée sur la figure 2.
